(19) Europäisches Patentamt
European Patent Office
Office européen des brevets

(11) Veröffentlichungsnummer: **0 119 949**
**B1**

(12) # EUROPÄISCHE PATENTSCHRIFT

(45) Veröffentlichungstag der Patentschrift: **23.11.88**

(51) Int. Cl.⁴: **C 07 C 177/00**, A 61 K 31/557

(21) Anmeldenummer: **84730009.2**

(22) Anmeldetag: **09.02.84**

(54) **Neue Carbacycline, Verfahren zu ihrer Herstellung und ihre Verwendung als Arzneimittel.**

(30) Priorität: **18.02.83 DE 3306123**

(43) Veröffentlichungstag der Anmeldung: **26.09.84 Patentblatt 84/39**

(45) Bekanntmachung des Hinweises auf die Patenterteilung: **23.11.88 Patentblatt 88/47**

(84) Benannte Vertragsstaaten: **AT BE CH DE FR GB IT LI LU NL SE**

(56) Entgegenhaltungen:
**EP-A-0 055 208**
**EP-A-0 069 692**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 23, D-1000 Berlin 28 (DE)**
Erfinder: **Radüchel, Bernd, Dr., Gollanczstrasse 132, D-1000 Berlin 28 (DE)**
Erfinder: **Vorbrüggen, Helmut, Prof., Wilkestrasse 7, D-1000 Berlin 27 (DE)**
Erfinder: **Stürzebecher, Claus- Steffen, Dr., Kuckucksweg 6, D-1000 Berlin 33 (DE)**
Erfinder: **Haberey, Martin, Dr., Neckarsulmer Strasse 15, D-1000 Berlin 46 (DE)**
Erfinder: **Schillinger, Ekkehard, Dr., Im Amseltal 50, D-1000 Berlin 28 (DE)**
Erfinder: **Town, Michael Harold, Dr., Zugspitzstrasse 13, D-8127 Iffeldorf (DE)**

**EP 0 119 949 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft (5E)-13,14,18,18,19,19-Hexadehydro-3-oxa-6a-carba-prostaglandin-$I_2$-derivate, deren physiologisch verträglichen Salze, Verfahren zu ihrer Herstellung und ihre pharmazeutische Anwendung.

In der DE-A-3 048 906.6 werden Carbacyclinderivate der allgemeinen Formel

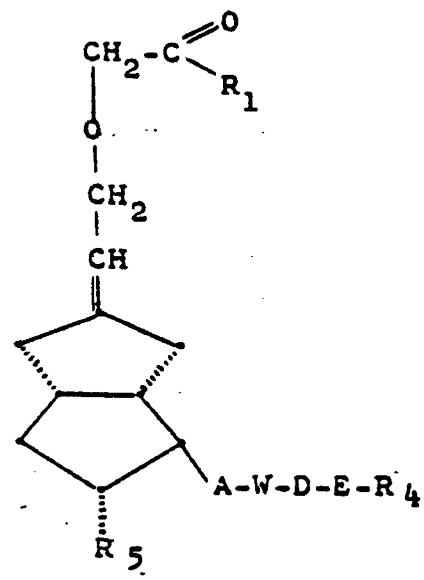

beansprucht, in der

$R_1$ den Rest $OR_2$, wobei $R_2$ Wasserstoff, Alkyl, Cycloalkyl, Aryl oder einen heterocyclischen Rest bedeuten kann, oder den Rest $NHR_3$ mit $R_3$ in der Bedeutung eines Säurerestes oder Wasserstoffatoms,

A eine $-CH_2-CH_2-$, trans-$CH=CH$- oder $C \equiv C$-Gruppe,

W eine freie oder funktionell abgewandelte Hydroxymethylengruppe oder eine freie oder funktionell abgewandelte

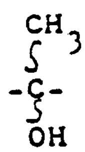 Gruppe, wobei die OH-Gruppe α- oder β-ständig sein kann,

D eine geradkettige oder verzweigte, gesättigte oder ungesättigte Alkylengruppe mit 1 - 10 C-Atomen, die gegebenenfalls durch Fluoratome substituiert sein kann,

E eine $-C \equiv C$-Bindung oder eine $-CR_6=CR_7$-Gruppe darstellt, wobei $R_6$ und $R_7$ sich unterscheiden und ein Wasserstoffatom oder eine Alkylgruppe mit 1 - 5 C-Atomen bedeuten,

$R_4$ eine Alkyl-, Cycloalkyl-, oder eine gegebenenfalls substituierte Aryl- oder eine heterocyclische Gruppe,

$R_5$ eine freie oder funktionell abgewandelte Hydroxygruppe, und falls $R_2$ die Bedeutung eines Wasserstoffatoms hat, deren Salze mit physiologisch verträglichen Basen bedeuten.

Die Verbindungen besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprosektion und damit Senkung des systemischen Blutdrucks, ohne zugleich Schlagvolumen und koronare Durchblutung zu senken, Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion, Zytoprotektion der Magen- und Darmschleimhaut, antiallergische Eigenschaften, Senkung des pulmonaren vaskulären Widerstandes und des pulmonaren Blutdrucks, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Prostaglandinanaloga antiproliferative Eigenschaften.

Unter den in der DE-A-3 048 906.6 beanspruchten Verbindungen zeigten die (5E)-13,14,18,18,19,19-Hexadehydro-3-oxa-6a-carba-prostaglandin-$I_2$-Verbindungen als blutdrucksenkende Mittel und Thrombozytenaggregationshemmende Mittel derartig herausragende Eigenschaften, daß damit die Dosierung weiter herabgesetzt werden kann, wodurch auch unerwünschte Nebenwirkungen noch stärker zurückgedrängt werden. Die (5E)-13,14,18,18,19,19-Hexadehydro-3-oxa-6a-carba-prostaglandin-$I_2$-Verbindungen sind in der DE-A-3 048 906.6 nicht namentlich beschrieben. Verbindungen mit A als $-C \equiv C$-Gruppe werden gegenüber den

anderen Verbindungen, in denen A eine $-CH_2-CH_2-$ oder trans-CH = CH-Gruppe bedeutet, nicht herausgestellt.

Die Nomenklatur der Verbindungen basiert auf einem Vorschlag von Morton und Brokaw (J. Org. Chem., 44, 2280 [1979]). (5E)-6a-Carba-prostaglandin-$I_2$ hat danach folgende Strukturformel:

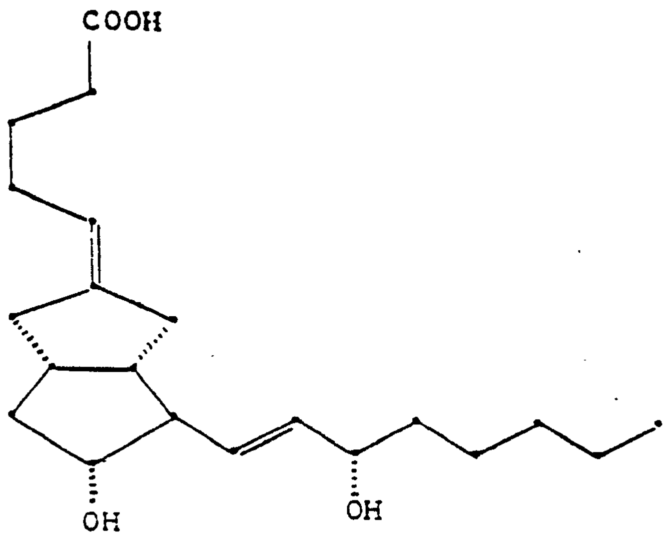

Die Erfindung betrifft somit (5E)-13,14,18,18,19,19-Hexadehydro-3-oxa-6a-carba-prostaglandin-$I_2$-Derivate der allgemeinen Formel I

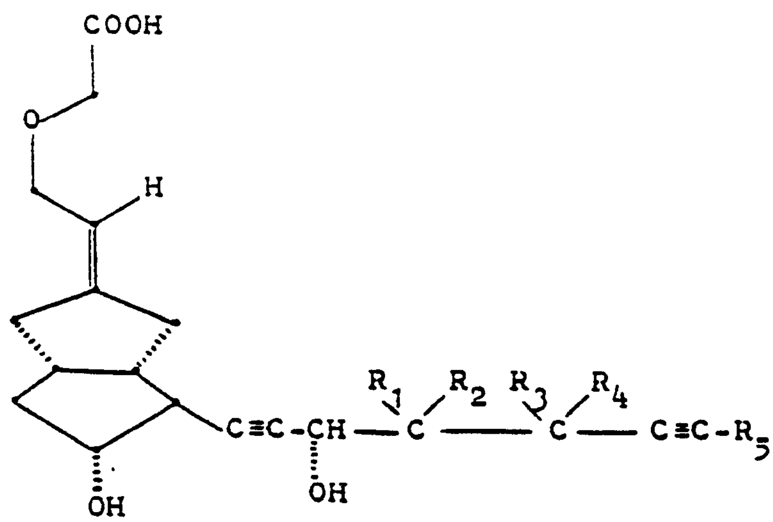

(I),

worin

$R_1$, $R_2$, $R_3$, $R_4$ ein Wasserstoffatom oder eine Alkylgruppe mit 1 - 5 C-Atomen,

$R_5$ eine Alkylgruppe mit 1 - 5 C-Atomen bedeuten, sowie deren Salze mit physiologisch verträglichen Basen.

Als Alkylgruppen $R_1$, $R_2$, $R_3$, $R_4$, $R_5$ kommen gerad- und verzweigtkettige Alkylreste mit 1 - 5 Kohlenstoffatomen in Betracht wie beispielsweise Methyl, Äthyl, Propyl, Butyl, Isopropyl, Isobutyl, Pentyl. Bevorzugte Reste sind Methyl, Äthyl, Propyl und Isopropyl, insbesondere Methyl und Äthyl.

Zur Salzbildung sind anorganische und organische Basen geeignet, wie sie dem Fachmann zur Bildung physiologisch verträglicher Salze bekannt sind. Beispielsweise genannt seien Alkalihydroxide, wie Natrium- und Kaliumhydroxid, Erdalkalihydroxid wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen Prostacyclinderivate der allgemeinen Formel I, dadurch gekennzeichnet, daß man eine Verbindung der allgemeinen Formel II

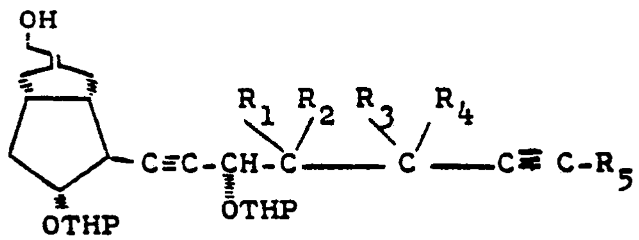

(II),

worin $R_1$, $R_2$, $R_3$, $R_4$, $R_5$, die oben angegebenen Bedeutungen aufweisen und THP den Tetrahydropyranylrest bedeutet mit einem Halogenessigsäurederivat der allgemeinen Formel III,

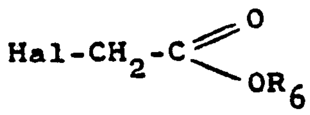

( III ),

wobei Hal ein Chlor-, Brom- oder Jodatom und $R_6$ einen $C_1$-$C_4$-Alkylrest oder Trialkyl-silylrest mit $C_1$-$C_4$-Alkylgruppen oder ein Alkalimetall (Na, Li, K) bedeutet, in Gegenwart einer Base veräthert und gegebenenfalls anschließend in beliebiger Reihenfolge Ester verseift Isomere trennt, Schutzgruppen abspaltet und gegebenenfalls die Carboxylgruppe mit einer physiologisch verträglichen Base in ein Salz überführt.

Die Umsetzung der Verbindung der allgemeinen Formel II mit einem Halogenessigsäurederivat der allgemeinen Formel III wird bei Temperaturen von 0°C bis 100°C, vorzugsweise 10°C bis 80°C, in einem aprotischen Lösungsmittel oder Lösungsmittelgemisch, beispielweise Dimethylsulfoxid, Dimethylformamid, Tetrahydrofuran usw. vorgenommem. Als Basen kommen die dem Fachmann für Verätherungen bekannte Basen in Frage, beispielsweise Natriumhydrid, Kalium-tert.-butylat, Butyllithium usw. Die Verseifung der Carbacyclinester wird nach den dem Fachmann bekannten Methoden durchgeführt, wie beispielweise mit basischen Katalysatoren.

Die Schutzgruppenabspaltung wird in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesatzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 30°C durchgeführt.

Die Carbonsäuren der allgemeinen Formel I können mit geeigneten Mengen der entsprechenden anorganischen Basen unter Neutralisierung in Salze überführt werden. Beispielsweise erhält man beim Lösen der entsprechenden Säuren in Wasser, welches die stöchiometrische Menge Base enthält, nach Abdampfen des Wassers oder nach Zugabe eines mit Wasser mischbaren Lösungsmittels, zum Beispiel Alkohol oder Aceton, das feste anorganische Salz. Zur Herstellung eines Aminsalzes wird die PG-Säure in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die als Ausgangsmaterial dienenden Verbindungen der allgemeinen Formel II können beispielsweise hergestellt werden, indem man in an sich bekannter Weise einen Aldehyd der Formel IV (DE-A-2 845 770)

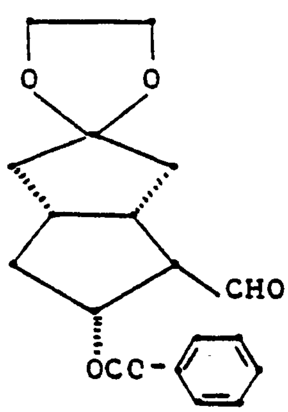

(IV)

mit einem Phosphonat der allgemeinen Formel V

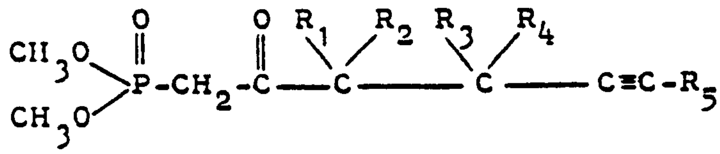

(V)

worin $R_1$, $R_2$, $R_3$, $R_4$ und $R_5$ die oben angegebenen Bedeutungen aufweisen, in Gegenwart eines Deprotonierungsmittels, wie beispielsweise Natriumhydrid oder Kalium-tert.-butylat und eines Bromierungsmittels, wie beispielsweise N-Bromsuccinimid, zu einem Keton der allgemeinen Formel VI umsetzt und gegebenenfalls anschließend Diastereomere trennt.

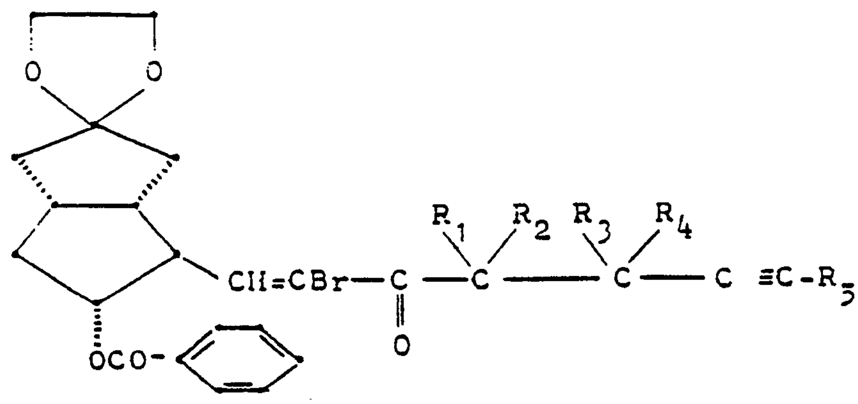

(VI)

Nach Reduktion der Ketogruppe mit Natriumborhydrid und gegebenenfalls Diastereomerentrennung, Verseifung der Estergruppe beispielweise mit Kaliumcarbonat und gegebenenfalls Diastereomerentrennung, Verätherung mit Dihydropyran und anschließende Abspaltung von Bromwasserstoff mit beispielweise Kalium-tert.-butylat in Dimethylsulfoxid gelangt man zur Acetylenverbindung der allgemeinen Formel VII

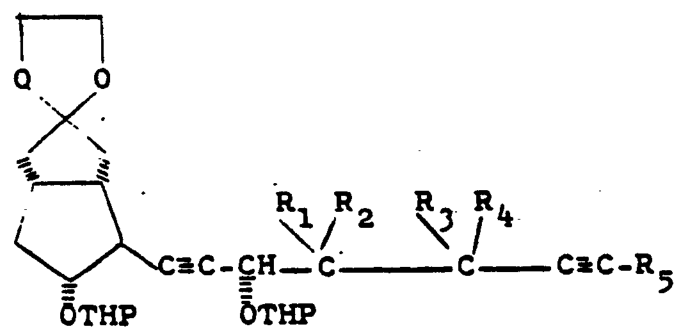

( VII )

Behandlung mit, wässriger Essigsäure sowie gegebenenfalls Diastereomerentrennung und anschließende Verätherung mit Dihydropyran liefert das Keton der allgemeinen Formel VIII.

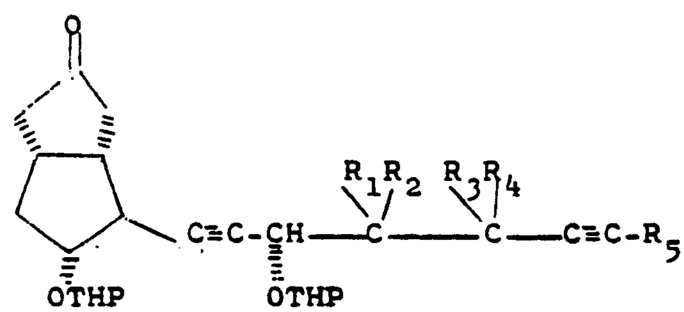

(VIII)

Nach Olefinierungsreaktion mit Phosphonoessigsäuretriäthylester oder Phosphonoessigsäuretrimethylester, Reduktion mit Lithiumaluminiumhydrid und anschließender Trennung der Doppelbindungsisomeren erhält man die Verbindungen der allgemeinen Formel II.

Die Herstellung der Phosphonate der allgemeinen Formel V erfolgt in an sich bekannter Weise durch Umsetzung eines Alkylhalogenids (herstellbar aus dem entsprechenden Alkohol durch Halogenierung) der allgemeinen Formel IX

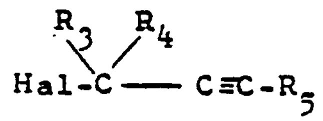

(IX)

mit dem aus dem Phosphonat der allgemeinen Formel X erzeugten Dianions,

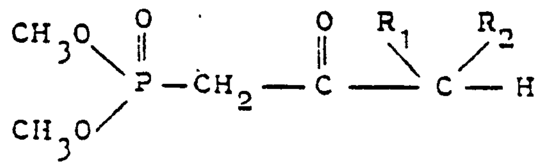

(X)

worin $R_1$, $R_2$, $R_3$, $R_4$ und $R_5$ die oben angegebenen Bedeutungen aufweisen.

Einen weiteren Zugang zu den Phosphonaten der allgemeinen Formel V besteht in der Umsetzung des Anions von Methylphosphonsäuredimethylester mit einem Ester der allgemeinen Formel XI

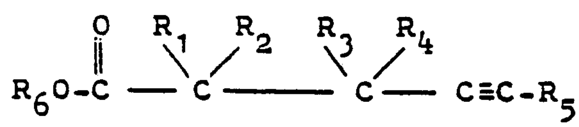

(XI)

worin $R_1$, $R_2$, $R_3$, $R_4$ und $R_5$ die oben angegebenen Bedeutungen aufweisen und $R_6$ eine Alkylgruppe mit 1 - 5 C-Atomen bedeutet, den man aus dem entsprechenden Malonsäureester durch Alkylierung mit dem Halogenid der allgemeinen Formel IX und anschließender Decarbalkoxylierung erhalten kann. Der Ester der allgemeinen Formel XI ist auch aus der Carbonsäure der allgemeinen Formel XII

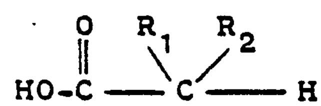

( XII )

worin $R_1$ und $R_2$ die oben angegebenen Bedeutungen aufweisen durch Alkylierung mit dem Halogenid der allgemeinen, Formel IX und anschließender Veresterung zugänglich.

Die Verbindungen dieser Erfindung wirken blutdrucksenkend und bronchodilatorisch. Sie sind weiterhin geeignet zur Hemmung der Thrombozyten-Aggregation. Folglich stellen die neuen Prostacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Derivate besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdrucks ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen- und Darmschleimhaut, Zytoprotektion in der Leber und im Pankreas; antiallergische Eigenschaften, Senkung des pulmonaren vaskulären Widerstandes und des pulmonaren Blutdrucks, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Carbacyclin-Derivate antiproliferative Eigenschaften. Die Carbacycline dieser Erfindung können auch in Kombination, z. B. mit β-Blockern oder Diuretika, verwendet werden.

Die Dosis de Verbindungen ist 1 - 1500 µg/kg/Tag, wenn sie am menschlichen Patienten verabreicht werden. Die Einheitsdosis für den pharmazeutischen akzeptablen Träger beträgt 0,01 - 100 mg.

Bei intravenöser Injektion an wachen, hypertonen Ratten in Dosen von 5, 20 und 100 µg/kg Körpergewicht

zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen im Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wäßrige oder ölige Lösungen benutzt. Für die orale Applikation sind beispielsweise Tabletten, Dragées oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z. B. zur Herstellung von Blutdrucksenkern dienen.

**Beispiel 1**

(5E)-(16S)-16,20-Dimethyl-3-oxa-13,14,18,18,19,19-hexadehydro-6a-carba-prostaglandin $I_2$

Zu einer Lösung von 0,4 g 2-{(E)-(1S,5S,6S,7R)-7-(Tetrahydropyran-2-yloxy)-6-[(3S,4S)-4-methyl-3-(tetrahydropyran-2-yloxy)-nona-1,6-diinyl]-bicyclo[3.3.0]-octan-3-yliden}-äthan-1-ol in 12 ml Tetrahydrofuran fügt man 80 mg 55-%-ige Natriumhydridsuspension (in Minieralöl) und kocht 1 Stunde am Rückfluß. Man fügt eine Lösung von 127 mg Bromessigsäure in 4 ml Tetrahydrofuran zu, kocht 18 Stunden am Rückfluß, verdünnt mit Äther und schüttelt viermal mit je 30 ml 5-%-iger Natronlauge. Dieser Extrakt wird mit 10-%-iger Schwefelsäure bei 0°C auf $pH_3$ eingestellt und mit Methylenchlorid extrahiert. Der organische Extrakt wird mit Sole geschüttelt, über Magnesiumsulfat getrocknet und im Vakuum eingedampft. Man erhält 220 mg (5E)-(16S)-16,20-Dimethyl-3-oxa-13,14,18,18,19,19-hexadehydro-6a-carba-prostaglandin-$I_2$-11,15-bis-(tetrahydropyranyläther), die man zur Abspaltung der Schutzgruppen 18 Stunden mit 15 ml Essigsäure/Wasser/Tetrahydrofuran (65/35/10) bei 25°C rührt. Man dampft unter Zusatz von Toluol ein und chromatographiert den Rückstand an Kieselgel mit Essigester / 0,1 - 1 % Essigsäure. Dabei erhält man 145 mg der Titelverbindung als farbloses Öl.

IR ($CHCl_3$): 3600, 3400 (breit), 2930, 2223, 1730, 1600, 1425, 1380/cm.

Das Ausgangsmaterial für die obige Titelverbindung wird wie folgt hergestellt:

1a) (1R,5S,6S,7R)-3,3-Äthylendioxy-7-benzoyloxy-6-[(4S)-2-brom-4-methyl-3-oxo-non-1-en-6-inyl]-bicyclo[3.3.0]-octan

Zu einer Suspension von 3,57 g Natriumhydrid (55 % in Mineralöl) in 360 ml Dimethoxyäthan tropft man bei 0°C eine Lösung von 21,9 g 3-Methyl-2-oxo-oct-5-in-phosphonsäuredimethylester in 140 ml Dimethoxyäthan, rührt 1 Stunde bei 0°C und fügt dann 14,56 g fein gepulvertes N-Bromsuccinimid hinzu. Man rührt 1 Stunde bei 0°C, versetzt mit einer Lösung aus 22,5 g (1R,5S,6R,7R)-3,3-Äthylendioxy-7-benzoyloxy-6-formyl-bicyclo[3.3.0]-octan in 180 ml Dimethoxyäthan und rührt 4 Stunden bei 0°C. Das Reaktionsgemisch wird mit 3 l Äther verdünnt, mit Sole neutral gewaschen, mit Natriumsulfat getrocknet und im Vakuum eingedampft. Der Rückstand wird mit Hexan/Äther als Elutionsmittel an Kieselgel chromatographiert. Nach dreimaliger Chromatographie der jeweiligen diastereomeren Mischfraktionen erhält man als polarere Verbindung 8,1 g (1R,5S,6S,7R)-3,3-Äthylendioxy-7-benzoyloxy-6-[(4R)-2-brom-4-methyl-3-oxo-non-1-en-6-inyl]-bicyclo[3.3.0]-octan und als unpolarere Verbindung 7,4 g der Titelverbindung als farblose Öle.

IR: 2935, 2878, 1715, 1690, 1601, 1595, 1450, 1270, 948/cm.

1b) (1R,5S,6S,7R)-3,3-Äthylendioxy-7-(tetrahydropyran-2-yloxy)-6-[3S,4S)-2-brom-4-methyl-3-(tetrahydropyran-2-yloxy)-non-1-en-6-inyl]-bicyclo[3.3.0]-octan

Zu einer Lösung von 7,4 g des nach Beispiel 1a) hergestellten Ketons in 140 ml Methanol fügt man bei -20°C portionsweise 3 g Natriumborhydrid und rührt 30 Minuten bei - 20°C. Anschließend verdünnt man mit Äther, wäscht mit Wasser neutral, trocknet über Magnesiumsulfat und dampft im Vakuum ein.

Das Rohprodukt (15-Epimerengemisch) löst man in 300 ml Methanol, fügt 2,95 g Kaliumcarbonat zu und rührt 21 Stunden bei 23°C unter Argon. Anschließend engt man im Vakuum ein, verdünnt mit Äther und wäscht mit Sole neutral. Man trocknet über Magnesiumsulfat und dampft im Vakuum ein. Durch Säulenchromatographie an Kieselgel mit Äther/Methylenchlorid (7+3) erhält man zunächst 2,6 g des 15 β-konfigurierten Alkohols sowie als polarere Komponente 2,1 g des 15α-konfigurierten Alkohols (PG-Nomenklatur) als farblose Öle.

Eine Lösung aus 2,1 g des vorstehend hergestellten 15α-Alkohols, 20 mg p-Toluolsulfonsäure und 1,4 g Dihydropyran in 50 ml Methylenchlorid rührt man 30 Minuten bei 0°C. Anschließend gießt man auf verdünnte Natriumbicarbonatlösung, extrahiert mit Äther, wäscht mit Wasser neutral, trocknet über Magnesiumsulfat und dampft im Vakuum ein. Nach Chromatographie des Rückstandes an Kieselgel erhält man mit Hexan/Äther (6+4) 2,6 g der Titelverbindung als farbloses Öl.

IR: 2939, 2577, 1450, 969, 948 / cm.

1c) (1R,5S,6S,7R)-7-(Tetrahydropyran-2-yloxy)-6-[(3S,4S)-4-methyl-3-(tetrahydropyran-2-yloxy)-nona-1,6-diinyl]-bicyclo[3.3.0]-octan-3-on

Eine Lösung von 290 mg der nach Beispiel 1b) hergestellten Verbindung in 2,5 ml Dimethylsulfoxid und 1 ml Tetrahydrofuran versetzt man mit 112 mg Kalium-tert.-Butylat und rührt 2 Stunden bei 23°C. Man verdünnt mit 10 ml Wasser und extrahiert dreimal mit je 10 ml Äther/Hexan (7+3), wäscht den Extrakt mit Wasser neutral, trocknet über Sole und dampft im Vakuum ein.

Man rührt den Rückstand 22 Stunden mit 15 ml Essigsäure/Wasser/Tetrahydrofuran (65/35/10), dampft im Vakuum unter Zusatz von Toluol ein und reinigt den Rückstand durch Chromatographie an Kieselgel. Mit Äther eluiert man 150 mg ölige Substanz, die man in 5 ml Dichlormethan mit 140 mg Dihydropyran und 1 mg p-Toluolsulfonsäure bei 0°C umsetzt. Nach 30 Minuten verdünnt man mit Äther, schüttelt mit 5-%-iger Natriumbicarbonatlösung und Sole, trocknet über Magnesiumsulfat und dampft im Vakuum ein. Nach Chromatographie des Rückstandes an Kieselgel mit Hexan/Äther (1+1) erhält man 185 mg der Titelverbindung als farbloses Öl.

IR: 2940, 2876, 2216, 1738, 1020, 970 / cm.

1d) 2-{(E)-(1S,5S,6S,7R)-7-(Tetrahydropyran-2-yloxy)-6-[(3S,4S)-4-methyl-3-(tetrahydropyran-2-yloxy)-nona-1,6-diinyl]-bicyclo[3.3.0]-octan-3-yliden}-äthan-1-ol

Zu einer Lösung von 529 mg Phosphonoessigsäuretriäthylester in 10 ml Tetrahydrofuran fügt man bei 0°C 225 mg Kalium-tert.-butylat, rührt 10 Minuten, versetzt mit einer Lösung aus 0,6 g des nach Beispiel 1c) hergestellten Ketons in 6 ml Toluol und rührt 22 Stunden bei 23°C. Man verdünnt mit 150 ml Äther, schüttelt einmal mit Wasser, einmal mit 20-%-iger Natronlauge, wäscht mit Wasser neutral, trocknet über Magnesiumsulfat und dampft im Vakuum ein. Den Rückstand filtriert man mit Hexan/Äther (6+4) über Kieselgel. Dabei erhält man 0,58 g des ungesättigten Esters als farbloses Öl.

IR: 2940, 2670, 2212, 1704, 1655, 970/cm.

Man fügt 150 mg Lithiumaluminiumhydrid portionsweise bei 0°C zu einer gerührten Lösung von 570 mg des vorstehend hergestellten Esters in 25 ml Äther und rührt 30 Minuten bei 0°C. Man zerstört den Reagenzüberschuß durch tropfenweise Zugabe von Essigester, fügt 1 ml Wasser zu, rührt 3 Stunden bei 20°C, filtriert und dampft im Vakuum ein. Den Rückstand chromatographiert man mit Äther/Hexan (3+2) an Kieselgel. Dabei erhält man als unpolarere Verbindung 140 mg 2-{(Z)-(1S,5S,6S,7R)-7-(Tetrahydropyran-2-yloxy)-6-[(3S,4S)-4-methyl-3-(tetrahydropyran-2-yloxy)-nona-1,6-diinyl]-bicyclo[3.3.0]-octan-3-yliden}-äthan-1-ol und 180 mg der Titelverbindung als farbloses Öl.

IR: 3620, 3450 (breit), 2940, 2860, 2212, 970/cm.

**Beispiel 2**

(5E)-(16R)-16,20-Dimethyl-3-oxa-13,14,18,18,19,19-hexadehydro-6a-carba-prostaglandin-$I_2$

In Analogie zu Beispiel 1 erhält man aus 0,6 g 2-{(E)-(1S,5S,6S,7R)-7-(Tetrahydropyran-2-yloxy)-6-[(3S,4R)-4-methyl-3-(tetrahydropyran-2-yloxy)-nona-1,6-diinyl]-bicyclo[3.3.0]-octan-3-yliden}-äthan-1-ol 0,26 g der Titelverbindung als farbloses Öl.

IR: 3600, 3410 (breit), 2930, 2222, 1730, 1600, 1425, 1380/cm.

Das Ausgangsmaterial für die obige Titelverbindung wird wie folgt hergestellt:

2a) (1R,5S,6S,7R)-3,3-Äthylendioxy-7-(tetrahydropyran-2-yloxy)-6-[3S,4R)-2-brom-4-methyl-3-(tetrahydropyran-2-yloxy)-non-1-en-6-inyl]-bicyclo[3.3.0]-octan

In Analogie zu Beispiel 1b) erhält man aus 8 g (1R,5S,6S,7R)-3,3-Äthylendioxy-7-benzoyloxy-6-[(4R)-2-brom-4-methyl-3-oxo-non-1-en-6-inyl]-bicyclo[3.3.0]-octan (polares 16-Methyldiastereomeres aus Beispiel 1a)) 2,9 g der Titelverbindung als farbloses Öl.

IR: 2940, 2878, 1450, 970, 948/cm.

2b) (1R,5S,6S,7R)-7-(Tetrahydropyran-2-yloxy)-6-[(3S,4R)-4-methyl-3-(tetrahydropyran-2-yloxy)-nona-1,6-diinyl]-bicyclo[3.3.0]-octan-3-on

In Analogie zu Beispiel 1c) erhält man aus 2,8 g der nach Beispiel 2a) hergestellten Verbindung 1,7 g der Titelverbindung als farbloses Öl.

IR: 2940, 2875, 2215, 1738, 1021, 970/cm.

2c) 2-{(E)-(1S,5S,6S,7R)-7-(Tetrahydropyran-2-yloxy)-6-[(3S,4R)-4-methyl-3-(tetrahydropyran-2-yloxy)-nona-1,6-diinyl]-bicyclo[3.3.0]-octan-3-yliden}-äthan-1-ol

In Analogie zu Beispiel 1d) erhält man aus 1,6 g des nach Beispiel 2b) hergestellten Ketons nach chromatographischer Isomerentrennung 0,4 g 2-{(Z)-(1S,5S,6S,7R)-7-(Tetrahydropyran-2-yloxy)-6-[(3S,4R)-4-methyl-3-(tetrahydropyran-2-yloxy)-nona-1,6-diinyl]-bicyclo[3.3.0]-octan-3-yliden}-äthan-1-ol und als polarere Komponente 0,5 g der Titelverbindung als farbloses Öl.
IR: 3600, 3440 (breit), 2942, 2860, 2212, 970/cm.

**Beispiel 3**

(5E)-(16RS)-16-Methyl-3-oxa-13,14,18,18,19,19-hexadehydro-6a-carba-prostaglandin-$I_2$

In Analogie zu Beispiel 1 erhält man aus 0,45 g 2-{(E)-(1S,5S,6S,7R)-7-(Tetrahydropyran-2-yloxy)-6-[(3S,4RS)-4-methyl-3-(tetrahydropyran-2-yloxy)-octa-1,6-diinyl]-bicyclo[3.3.0]-octan-3-yliden}-äthan-1-ol 0,2 g der Titelverbindung als farbloses Öl.
IR: 3610, 3400 (breit), 2932, 2221, 1730, 1600/cm.
Das Ausgangsmaterial für die obige Titelverbindung wird wie folgt hergestellt:

3a) (1R,5S,6S,7R)-3,3-Äthylendioxy-7-benzoyloxy-6-[(4RS)-2-brom-4-methyl-3-oxo-oct-1-en-6-inyl]-bicyclo[3.3.0]-octan

Zu einer Suspension von 1,81 g Natriumhydrid in 180 ml Dimethoxyäthan tropft man bei 0°C eine Lösung von 10,5 g 3-Methyl-2-oxo-hept-5-in-phosphonsäuredimethylester in 70 ml Dimethoxyäthan, rührt 1 Stunde bei 0°C und fügt dann 7,4 g fein gepulvertes N-Bromsuccinimid hinzu. Man rührt 30 Minuten bei 0°C, versetzt mit einer Lösung von 11,4 g (1R,5S,6R,7R)-3,3-Äthylendioxy-7-benzoyloxy-6-formyl-biyclo[3.3.0]-octan in 90 ml Dimethoxyäthan und rührt 2 Stunden bei 0°C. Das Reaktionsgemisch gießt man auf gesättigte Ammoniumchloridlösung und extrahiert mit Äther. Man wäscht den organischen Extrakt mit Wasser neutral, trocknet über Magnesiumsulfat und dampft im Vakuum ein. Nach Chromatographie des Rückstandes an Kieselgel erhält man mit Hexan/Äther (3+2) 8,2 g des ungesättigten Ketons als farbloses Öl.
IR: 2930, 2880, 1712, 1688, 1602, 1595, 1450, 1275, 945 /cm.

3b) (1R,5S,6S,7R)-3,3-Äthylendioxy-7-(tetrahydropyran-2-yloxy)-6-[(3S,4RS)-2-brom-4-methyl-3-(tetrahydropyran-2-yloxy)-oct-1-en-6-inyl]-bicyclo[3.3.0]-octan

Zu einer Lösung von 5,9 g des nach Beispiel 3a) hergestellten Ketons in 140 ml Methanol fügt man bei - 40 °C portionsweise 2,5 g Natriumborhydrid und rührt 30 Minuten bei - 40°C. Anschließend verdünnt man mit Äther, wäscht mit Wasser neutral, trocknet über Magnesiumsulfat und dampft im Vakuum ein. Das Rohprodukt (15-Epimerengemisch), löst man in 200 ml Methanol, fügt 2,5 g Kaliumcarbonat zu und rührt 17 Stunden bei 23°C unter Argon. Anschließend engt man im Vakuum ein, verdünnt mit Äther und wäscht mit Sole neutral. Man trocknet über Magnesiumsulfat und dampft im Vakuum ein. Durch Säulenchromatographie an Kieselgel mit Äther/Methylenchlorid (7+3) erhält man zunächst 1,6 g des 15β-konfigurierten Alkohols sowie als polarere Komponente 2,1 g der Titelverbindung (PG-Nomenklatur 15α-Hydroxy) als farblose Öle. Eine Lösung aus 1,6 g des α-Alkohols, 16 mg p-Toluolsulfonsäure und 1,5 g Dihydropyran in 50 ml Methylenchlorid rührt man 35 Minuten bei 0°C.
Anschließend verdünnt man mit Äther, schüttelt mit verdünnter Natriumcarbonatlösung, wäscht mit Wasser neutral trocknet über Magnesiumsulfat und dampft im Vakuum ein. Nach Chromatographie des Rückstandes an Kieselgel erhält man mit Hexan/Äther (7+3) 2,17 g der Titelverbindung als farbloses Öl.
IR: 2940, 2870, 1450, 1120, 1018, 965, 948 /cm.

3c) (1R,5S,6S,7R)-7-(Tetrahydropyran-2-yloxy)-6-[(3S,4RS)-4-methyl-3-(tetrahydropyran-2-yloxy)-octa-1,6-diinyl]-bicyclo[3.3.0]-octan-3-on

Eine Lösung von 2,30 g der nach Beispiel 3b) hergestellten Verbindung in 23 ml Dimethylsulfoxid und 10 ml Tetrahydrofuran gibt man 667 mg Kalium-tert.- Butylat und rührt 2 Stunden bei 20°C. Man verdünnt mit 100 ml Wasser und extrahiert dreimal mit je 100 ml Äther/Hexan (8+2), wäscht den Extrakt mit je 30 ml Wasser und Sole, trocknet über Magnesiumsulfat und dampft im Vakuum ein. Man rührt den Rückstand 22 Stunden mit 75 ml Essigsäure/Wasser/Tetrahydrofuran (65/35/10), dampft im Vakuum ein und reinigt den Rückstand durch Chromatographie an Kieselgel. Mit Äther eluiert man 1,05 g ölige Substanz, die man in 40 ml Dichlormethan mit 0.91 g Dihydropyran und 10 mg p-Toluolsulfonsäure bei 0°C umsetzt. Nach 30 Minuten verdünnt man mit Äther, schüttelt mit Natriumhydrogencarbonatlösung und Sole, trocknet über Magnesiumsulfat und dampft im Vakuum ein. Nach Chromatographie an Kieselgel mit Hexan/Äther (1 : 1) erhält man 1,53 g der Titelverbindung als farbloses Öl.
IR: 2942, 2876, 2210, 1737, 1018, 970, 905, 868 /cm.

3d) 2-{(E)-(1S,5S,6S,7R)-7-(Tetrahydropyran-2-yloxy)-6-[(3S,4RS)-4-methyl-3-(tetrahydropyran-2-yloxy)-octa-

1,6-diinyl]-bicyclo[3.3.0]-octan-3-yliden}-äthan-1-ol

In Analogie zu Beispiel 1d) erhält man aus 1,4 g des nach Beispiel 3c) hergestellten Ketons nach chromatographischer Isomerentrennung 0,37 g 2-{(Z)-(1S,5S,6S,7R)-7-(Tetrahydropyran-2-yloxy)-6-[(3S,4RS)-4-methyl-3-(tetrahydropyran-2-yloxy)-octa-1,6-diinyl]-bicyclo[3.3.0]-octan-3-yliden}-äthan-1-ol und als polarere Komponente 0,48 g der Titelverbindung als farbloses Öl.

IR: 3600, 3420 (breit), 2945, 2860, 2225 /cm.

**Beispiel 4**

(5E)-(16S)-16,20-Dimethyl-3-oxa-13,14,18,18,19,19-hexadehydro-6a-carba-prostaglandin-$I_2$-tris-(hydroxymethyl)-aminomethan-salz

Zu einer Lösung von 55 mg (5E)-(16S)-16,20-Dimethyl-3-oxa-13,14,18,18,19,19-hexadehydro-6a-carba-prostaglandin-$I_2$ in 8 ml Acetonitril fügt man bei 68°C eine Lösung von 15 mg Tris-(hydroxymethyl)-aminomethan in 0,05 ml Wasser. Man läßt unter Rühren abkühlen, dekantiert nach 16 Stunden vom Lösungsmittel und trocknet den Rückstand im Vakuum. Man isoliert 40 mg der Titelverbindung als zähes Öl.

**Patentansprüche**

1. (5E)-13,14,18,18,19,19-Hexadehydro-3-oxa-6a-carba-prostaglandin-$I_2$-Derivate der allgemeinen Formel I

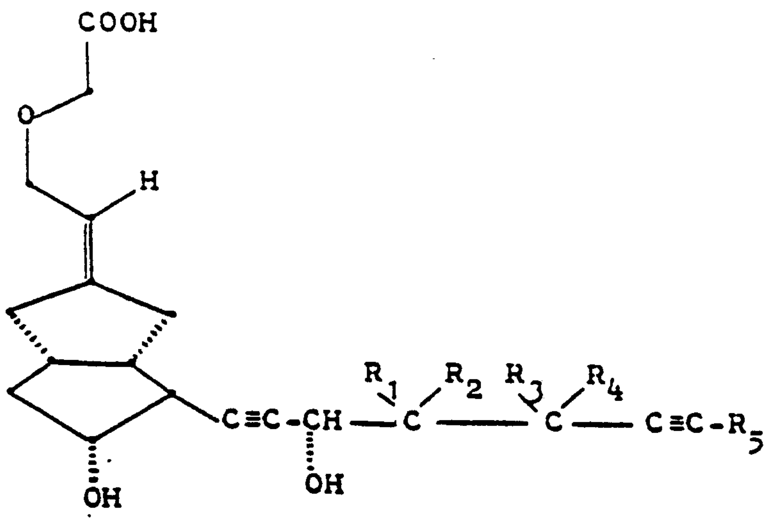

(I),

worin

$R_1$, $R_2$, $R_3$, $R_4$ ein Wasserstoffatom oder eine Alkylgruppe mit 1 - 5 C-Atomen,

$R_5$ eine Alkylgruppe mit 1 - 5 C-Atomen bedeuten, sowie deren Salze mit physiologisch verträglichen Basen.

2. Verfahren zur Herstellung der Carbacyclinderivate der allgemeinen Formel I, dadurch gekennzeichnet, daß man eine Verbindung der allgemeinen Formel II

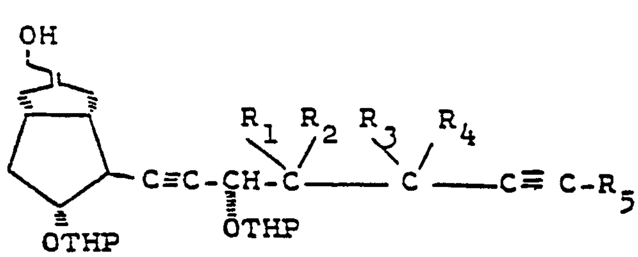

(II),

worin $R_1$, $R_2$, $R_3$, $R_4$, $R_5$, die oben angegebenen Bedeutungen aufweisen und THP den Tetrahydropyranylrest bedeutet mit einem Halogenessigsäurederivat der allgemeinen-Formel III,

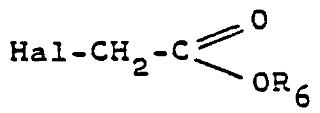

( III ),

wobei Hal ein Chlor-, Brom- oder Jodatom und $R_6$ einen $C_1$-$C_4$-Alkylrest oder Trialkyl-silylrest mit $C_1$-$C_4$-Alkylgruppen oder ein Alkalimetall (Na, Li, K) bedeutet, in Gegenwart einer Base veräthert und gegebenenfalls anschließend in beliebiger Reihenfolge Ester verseift Isomere trennt, Schutzgruppen abspaltet und gegebenenfalls die Carboxylgruppe mit einer physiologisch verträglichen Base in ein Salz überführt.

3. (5E)-(16S)-16,20-Dimethyl-3-oxa-13,14,18,18,19,19-hexadehydro-6a-carba-prostaglandin-$I_2$.

4. (5E)-(16R)-16,20-Dimethyl-3-oxa-13,14,18,18,19,19-hexadehydro-6a-carba-prostaglandin-$I_2$.

5. (5E)-(16RS)-16,20-Methyl-3-oxa-13,14,18,18,19,19-hexadehydro-6a-carba-prostaglandin-$I_2$.

6. (5E)-(16S)-16,20-Dimethyl-3-oxa-13,14,18,18,19,19-hexadehydro-6a-carba-prostaglandin-$I_2$-tris-(hydroxymethyl)-aminomethan-salz.

7. Arzneimittel, bestehend aus einer oder mehreren Verbindungen des Anspruchs 1 und üblichen Hilfs- und Trägerstoffen.

**Revendications**

1. Dérivés de l'hexahydro-13,14,18,18,19,19 oxa-3 carba-6a prostaglandine $I_2$ (5E) qui répondent à la formule générale I :

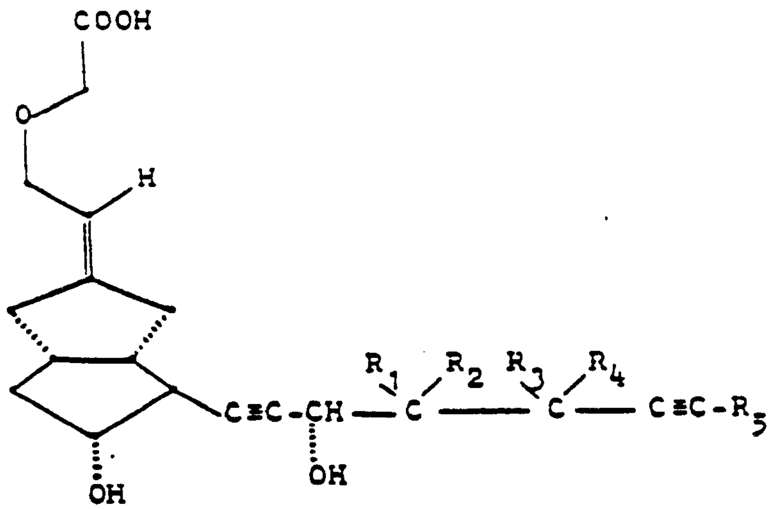

(I)

dans laquelle:

$R_1$, $R_2$, $R_3$ et $R_4$ représentent chacun un atome d'hydrogène ou un radical alkyle contenant de 1 à 5 atomes de carbone, et

$R_5$ représente un radical alkyle contenant de 1 à 5 atomes de carbone,

ainsi que les sels qu'ils forment avec des bases acceptables du point de vue physiologique.

2. Procédé pour préparer des carbacyclines de formule générale I, procédé caractérisé en ce qu'on éthérifie, en présence d'une base, un composé répondant à la formule générale II:

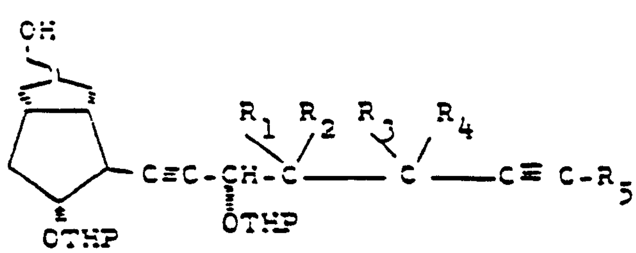

(II)

,

dans laquelle:

$R_1$, $R_2$, $R_3$, $R_4$ et $R_5$ ont les significations qui leur ont été données ci-dessus, et THP représente le radical tétrahydropyrannyle, avec un dérivé d'acide halogénoacétique répondant à la formule générale III:

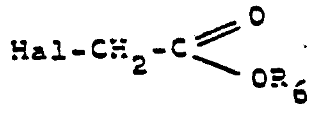

( III)

dans laquelle:

Hal représente un atome de chlore, de brome ou d'iode, et $R_6$ représente un radical alkyle en $C_1$-$C_4$, un radical trialkyl-sillyle dont chacun des alkyles contient de 1 à 4 atomes de carbone, ou un métal alcalin (Na, Li, K), puis, le cas échéant, en opérant dans l'ordre que l'on veut, on saponifie une fonction ester, on sépare des isomères, on élimine des radicaux protecteurs, et éventuellement on transforme le radical carboxy en un sel au moyen d'une base acceptable du point de vue physiologique.

3. Diméthyl-16,20 oxa-3 hexadéhydro-13,14,18,18,19,19 carba-6a prostaglandine $I_2$ (5E)-(16S).

4. Diméthyl-16,20 oxa-3 hexadéhydro-13,14,18,18,19,19 carba-6a prostaglandine $I_2$ (5E)-(16R).

5. Méthyl-16 oxa-3 hexadéhydro-13,14,18,18,19,19 carba-6a prostaglandine $I_2$ (5B)-(16RS).

6. Sel formé par la diméthyl-16,20 oxa-3 hexadéhydro-13,14,18,18,19,19 carba-6a prostaglandine $I_2$ (5E)-(16S) et le tris-(hydroxyméthyl)-aminométhane.

7. Médicament qui contient un ou plusieurs composés selon la revendication 1 ainsi que des adjuvants et excipients usuels.

## Claims

1. (5E)-13,14,18,18,19,19-hexadehydro-3-oxa-6a-carba-prostaglandin-$I_2$ derivatives of the general formula I

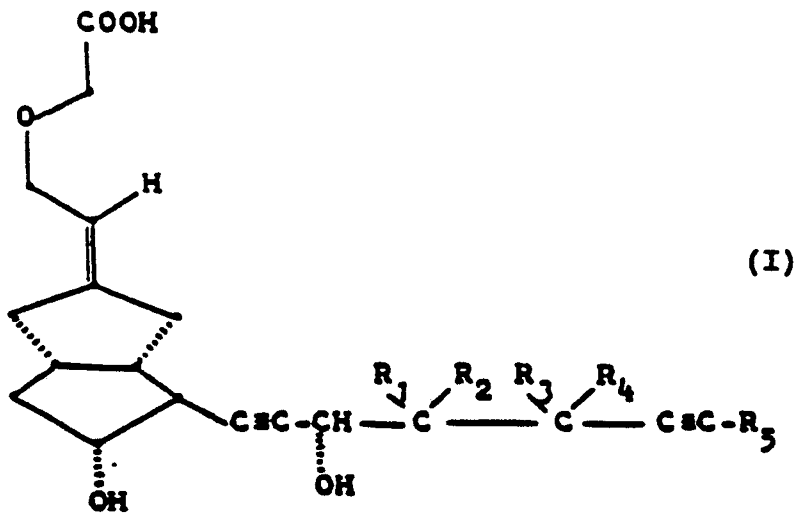

(I),

in which

$R_1$, $R_2$, $R_3$ and $R_4$ represent a hydrogen atom or an alkyl group having from 1 to 5 carbon atoms, and

$R_5$ represents an alkyl group having from 1 to 5 carbon atoms,

and salts thereof with physiologically acceptable bases.

2. Process for the manufacture of the carbacyclin derivatives of the general formula I, characterised in that a compound of the general formula II

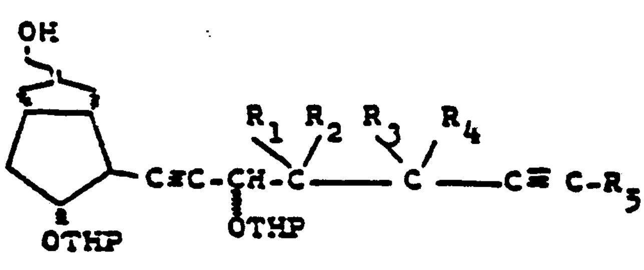

(II),

in which

$R_1$, $R_2$, $R_3$, $R_4$ and $R_5$ have the meanings given above and THP represents the tetrahydropyranyl radical, is etherified in the presence of a base with a haloacetic acid derivative of the general formula III

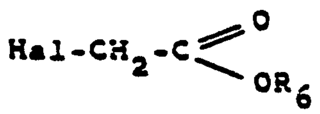 (III),

in which

Hal represents a chlorine, bromine or iodine atom and $R_6$ represents a $C_1$-$C_4$-alkyl radical, a trialkylsilyl radical having $C_1$-$C_4$-alkyl groups or an alkali metal (Na, Li, K), and then, optionally, in any desired order, esters are hydrolysed, isomers are separated, protecting groups are split off and, optionally, the carboxy group is converted into a salt with a physiologically acceptable base.

3. (5E)-(16S)-16,20-dimethyl-3-oxa-13,14,18,18,19,19-hexadehydro-6a-carba-prostaglandin-$I_2$.

4. (5E)-(16R)-16,20-dimethyl-3-oxa-13,14,18,18,19,19-hexadehydro-6a-carba-prostaglandin-$I_2$.

5. (5E)-(16RS)-16-methyl-3-oxa-13,14,18,18,19,19-hexadehydro-6a-carba-prostaglandin-$I_2$.

6. The salt of (5E)-(16S)-16,20-dimethyl-3-oxa-13,14,18,18,19,19-hexadehydro-6a-carba-prostaglandin-$I_2$-tris-(hydroxymethyl)-aminomethane.

7. Medicaments comprising one or more compounds of claim 1 and customary adjuncts and carriers.